# EUROPEAN PATENT SPECIFICATION

(11) **EP 4 108 672 B1**
(45) Date of publication and mention of the grant of the patent: **13.08.2025**
(21) Application number: 21756898.9
(22) Date of filing: 17.02.2021
(51) Int. Cl.: C07K 1/04, C40B 30/04, C40B 10/00

(54) **ANTIBODY SCREENING METHOD**
ANTIKÖRPER-SCREENING-VERFAHREN
PROCÉDÉ DE CRIBLAGE D'ANTICORPS

(30) Priority: 17.02.2020 CN 202010096612
(43) Date of publication of application: 28.12.2022
(73) Proprietor: Yichen Therapeutics Limited, Wan Chai, HK (HK)
(72) Inventor: WANG, Feng, Nantong, Jiangsu 226000 (CN); CHEN, Longxin, Nantong, Jiangsu 226000 (CN); LI, Xia, Nantong, Jiangsu 226000 (CN)
(74) Representative: V.O.
(86) International application number: PCT/CN2021/076666
(87) International publication number: WO 2021/164694

(56) References cited:
- WO-A2-2006/069203
- CN-A- 104 379 821
- CN-A- 110 187 122
- US-A1- 2009 036 315
- CHEN LONGXIN ET AL: "Epitope-directed antibody selection by site-specific photocrosslinking", SCI. ADV, 1 April 2020 (2020-04-01), XP093010589, Retrieved from the Internet <URL:https://www.ncbi.nlm.nih.gov/pmc/articles/PMC7112767/pdf/aaz7825.pdf> [retrieved on 20221222], DOI: 10.1126/sciadv.aaz7825
- RAY-SAHA SARMISTHA, HUBER THOMAS, SAKMAR THOMAS P.: "Antibody Epitopes on G Protein-Coupled Receptors Mapped with Genetically Encoded Photoactivatable Cross-Linkers", BIOCHEMISTRY, vol. 53, no. 8, 4 March 2014 (2014-03-04), pages 1302 - 1310, XP055839396, ISSN: 0006-2960, DOI: 10.1021/bi401289p
- THOMAS HUBER , THOMAS P. SAKMAR: "Probing Antibody Binding Sites on G Protein-Coupled Receptors Using Genetically Encoded Photo-Activatable Cross-Linkers", METHODS IN MOLECULAR BIOLOGY , vol. 1785, 13 September 2018 (2018-09-13), pages 65 - 75, XP009539513, ISSN: 1064-3745, DOI: 10.1007/978-1-4939-7841-0_5
- RUJAS EDURNE, CAAVEIRO JOSÉ M.M., INSAUSTI SARA, GARCÍA-PORRAS MIGUEL, TSUMOTO KOUHEI, NIEVA JOSÉ L.: "Peripheral Membrane Interactions Boost the Engagement by an Anti-HIV-1 Broadly Neutralizing Antibody", JOURNAL OF BIOLOGICAL CHEMISTRY, vol. 292, no. 13, 1 March 2017 (2017-03-01), US, pages 5571 - 5583, XP055839397, ISSN: 0021-9258, DOI: 10.1074/jbc.M117.775429

## Description

### Technical Field

The present disclosure relates to the selection and preparation of monoclonal antibodies.

### Background

Monoclonal antibodies have become a major source of drugs for treatment of various clinical indications. Traditional methods of obtaining monoclonal antibodies are generally through hybridoma or phage display technology. The epitope recognized by the monoclonal antibody is then determined by various methods such as protein truncation (generally used for linear epitopes) or phage display of peptide library (it may recognize spatial epitopes). The mouse hybridoma technology usually involves the following steps: immunizing mice with peptides or proteins, extracting spleen cells and fusing them with mouse myeloma cells to generate hybridomas, and finally selecting the hybridoma cells that can secrete antibodies with desired functions. In contrast, phage display technology can enrich and select candidate antibodies or n antibody fragments from large preconstructed antibody phage libraries by a plurality of rounds of panning against of the target antigens in vitro. Both methods are generally effective in obtaining antibodies with certain affinity.

Nonetheless, therapeutic antibodies often require bind to a specific epitope in an antigen to exert their functions, such as blocking ligand-receptor interactions, electing agonistic activities on receptors, and recruiting two proteins to form complexes. This epitope-dependence exerts a great challenge to existing selection methods. Although antibodies against linear epitopes can be obtained by synthesizing a short peptide and coupling macromolecules for immunization, it is difficult to select and obtain antibodies against spatially conformational epitopes due to the destruction of the spatial conformation of the antigen. Typically, one has to screen hundreds of hits phage panning or hybridoma clones generated from mouse immunization to hopefully identify the antibodies binding to the desire epitopes. Unfortunately, the epitopes on antigen are not equal in eliciting antibody responses. Antibodies produced from mouse immunization of a whole antigen are usually enriched to immune-dominant B cell epitopes, often diminishing the odds of identifying antibodies binding the desired epitopes with low response. Phage library screening technology based on binding affinities faces a similar epitope bias tissue, namely: antibody clones with high affinity to the antigen dominate the selection pool after several rounds of enrichment, thus limiting both the diversity of antibody sequences identified and the corresponding binding epitopes. Those hits with low to medium affinities are very likely masked by the strong binders, and are therefore difficult to be selected and are often lost during hit picking. In the discovery of monoclonal antibody drugs at present, it is important to retain more sequence diversity in the early stage of screening for the development of therapeutic antibodies, since affinity is only one of many criteria (physicochemical properties, stability, pharmacokinetics, immunogenicity and epitope specificity and the like) for selection of drug candidates. For this reason, one may intentionally decrease selection stringency to save those "weak hits" with weak functional binding. However, this may require further screening and optimum a large number of hits, which is not efficient if most of those hits do not bind to the target epitopes.

Despite great progress in antibody discovery technology development, there still exists the problem of epitope bias in affinity-based antibody screening, and there is still no simple solution to identifying the hits binding to specifically target epitopes.

The present invention relates to a method for selecting antibodies against a specific epitope of a target antigen described in the preamble of the independent claims. Relevant technologies are also known from Chen Longxin ET AL:"Epitope-directed antibody selection by site-specific photocrosslinking", Sci .Adv,1 April 2020 (2020-04-01).

### Summary

The purpose of the present disclosure is to provide a selection method that can overcome the epitope bias problem in antibody screening against target antigens, select antibodies against target epitopes, and improve sequence diversity of candidate antibodies in the antibody selection process.

The invention is set out in the appended set of claims 1-15.

In a general sense, the technical idea of the present disclosure is as follows: firstly, using the mutant antigen with incorporation of amino acids with photo cross-linking activities into or near a target epitope in the target antigen to screen antibody display library; secondly, applying appropriate light irradiation after the antibodies contact with the mutant antigen immobilized on a support so that the antibodies capable of binding to the target epitope form complexes with the mutant antigen through incorporated photo cross-linking amino acids due to the close vicinity between antibodies and mutant antigens, while antibodies that do not bind to the target epitope can't form such complexes since they are not close enough to the target epitope; then, elution is applied under certain conditions, which enable antibodies that do not covalently cross-link to the antigen to be washed away, while antibodies that are covalently cross-linked remain on the support. In this way, antibodies that can bind to the target epitope are selected from antibodies that cannot bind to the target epitope based on whether a covalent binding is formed, rather than the strength of binding affinity.

The noncanonical amino acids (ncAAs) p-benzoyl-L-phenylalanine (pBpa) and p-azido-L-phenylalanine (pAzF) have been incorporated into specific positions of proteins by genetic codon expansion method, and have been shown to covalently cross-link proteins of proximity upon ultraviolet (UV) irradiation. Researches have shown that the carbonyl of pBpa and the amino acids can cross-link with 3-12 Å of spatial distance between them, and with a wavelength of 365nm UV irradiation.

The inventor used the covalent cross-linking property of pBpa to incorporate pBpa into the epitope and its vicinity of human interleukin 1β (IL1β) protein through genetic codon expansion method. Then the inventors performed two rounds of panning for the target epitope and obtained more than one third of hits binding to the target epitope, of which there are clones that could cross-react with wild-type IL1β, thus verifying the idea of the present disclosure.

In order to verify the universality of the idea, this method was applied to another antigen human complement 5a (hC5a). By the panning of this strategy, half of the clones in the product bind to the target spatial conformational epitope which can distinguishanti-C5a from anti-C5 antibodies. By further affinity maturation, a monoclonal antibody with nano-molar affinity and selectivity against the hC5a target epitope was successfully selected. Subsequently, green fluorescence protein (GFP) expressed on the cell surface was taken as an example for selecting epitope-directed antibodies, and results showed that antibodies obtained by the selecting method of the present disclosure can cross-link the target epitope on GFP. Finally, this screening method was applied to select antibodies against multiple transmembrane protein adenylate receptor (A2A), and a series of antibodies that can cross-link with the target epitope of A2A were successfully obtained. Based on this, the present disclosure provides a method for selecting or isolating antibodies: amino acids with photo cross-linking activities (for example, by genetic codon expansion method) are incorporated into specific epitopes of the antigen, then the mutant antigen immobilized on the support contact with the antibody display library (for example, a phage display library) under appropriate irradiation condition. Antibodies covalently bound to mutant antigen could be isolated from other specific or non-specific antibodies that are not covalently bound to mutant antigen by specific elution condition (for example, competitive elution with wild-type target antigen-containing buffer followed by low-pH buffer).

The isolated antibodies were then subjected to digestion from the support to further select specific antibodies that are covalently bound with the ncAAs-containing epitopes. This selection can include repeated one or more rounds of the above-mentioned photo cross-linking selecting process, or can be carried out by a conventional selecting method based on affinity. Finally, the selected antibodies are further optimized for affinity maturation with wild-type antigens, and high-affinity monoclonal antibodies that can bind to the wild-type epitopes, namely epitope-specific antibodies, are selected. In addition, the antibody library that is covalently bound with the mutant antigen and the antibody library that is no covalently bound can also be used as panning pools, respectively.

For this reason, the present disclosure provides the following technical solutions.

In the first aspect, the present disclosure provides a method for selecting antibodies against a specific epitope of a target antigen, and the method comprises the followings steps:
(i) providing a support, herein the support is immobilized with a mutant antigen with incorporation of amino acids with photo cross-linking activity or its derivative in the target epitope or its vicinity;
(ii) providing conditions that enables the contact of the mutant antigen with the antibody display library, and applying light irradiation with suitable wavelength and energy to allow the mutant antigen to covalently cross-link with antibodies in the display library that binds to or near the target epitope to form an antigen-antibody complex;
(iii) performing elution under certain conditions, wherein this condition enables displayed antibodies that are not covalently cross-linked with the mutant antigen to be washed away from the support, while displayed antibodies that are covalently cross-linked remain on the support;
(iv) releasing the displayed antibodies that covalently cross-link with the mutant antigen from the support; and optionally;
(v) further selecting antibodies capable of binding to the target antigen from the displayed antibodies obtained in the step (iv).

In a specific embodiment, the amino acids with photo cross-linking activities or its derivative thereof is incorporated by genetic codon expansion. In certain embodiments, herein the step (v) includes repeating the steps (i)-(iv) for one or more rounds. In a specific embodiment, the method further includes sequencing the antibodies selected in the step (v). In certain embodiments, the epitope includes one or more amino acid residues.

In certain embodiments, the epitope is a linear epitope or a conformational epitope.

In a specific embodiment, herein the antibody display library is selected from: IgG antibodies or antibody fragments such as Fab libraries, single chain Fv (scFv) libraries, or nanobody libraries.

In a specific embodiment, the antibody display library is selected from: fully humanized antibody library, mouse immunized antibody library, humanized mouse immunized antibody library, alpaca immune nanobody library, and synthetic or semi-synthetic antibody library designed based on antibody sequences of different species.

In a specific embodiment, the display carrier of the antibody display library is selected from: phage, bacteria, yeast, or mammalian cells.

In a preferred embodiment, the antibody display library is a phage display antibody library. Traditional antibody phage library panning methods rely on the affinity of the phage-displayed antibodies for the whole antigen, and after multiple rounds of panning, those clones with high affinity are enriched and identified by sequencing. In the panning method for the antigen epitope-specific antibody of the present disclosure, the mutant antigen with incorporation of photo cross-linking amino acids can bind with the target epitope by forming covalent bonds with the antibody displayed on the support after UV irradiation in a certain wavelength range, while epitope non-specific phages may not be covalently cross-linked with the antigen and could be removed during vigorous washing steps such as acidic buffer. Therefore, on the one hand, only the antibody-displaying phages that bind to the target epitope can be enriched and selected; and on the other hand, in this process, the enrichment and panning of the antibody-displaying phages are not based on initial binding affinity but on the binding to target epitopes.

In a specific embodiment, the amino acid with photo cross-linking activity is a natural amino acid or a ncAA. In a further embodiment, the ncAA with photo cross-linking activity is selected from: p-benzoyl-L-phenylalanine (pBpa) or p-azido-L-phenylalanine (pAzF). The amino acids with photo cross-linking activity (such as pBpa) have a small molecular weight, and will not affect the spatial conformation of the antigen after being incorporated into a specific epitope. This is particularly important for the selection of conformational epitope-specific therapeutic antibodies.

In a specific embodiment, the light irradiation conditions suitable for pBpa crosslinking are: 365 nM wavelength and 6 W of energy. In a specific embodiment, the elution conditions of the step (iii) are selected from:
a) competitive elution with a buffer containing the target antigen;
b) acidic elution with low-pH buffer;
c) alkaline elution with a high-pH buffer.

In a specific embodiment, the cleavage g of the step (iv) is enzymatic digestion.

In a second aspect, the present disclosure provides a method for establishing an antibody panning pool, which includes the following steps:
(i) providing a support, herein the support is immobilized with a mutant antigen formed by incorporation of amino acids with photo cross-linking activity or its derivative thereof in the target epitope or its vicinity; (ii) providing conditions that enables the contact of the mutant antigen with the antibody display library, and applying ultraviolet irradiation with suitable wavelength and energy to allow the said mutant antigen to covalently cross-link antibodies in the library that binds to or near the target epitope to form an antigen-antibody complex;
(iii) performing elution under certain conditions, wherein this condition enables the antibodies that are not covalently cross-linked to the mutant antigen to be washed away from the support, while the displayed antibodies that are covalently cross-linked remains on the support;
(iv) releasing the displayed antibodies that covalently cross-link to the mutant antigen from the support;
(v) using the antibody library obtained in the step (iv) or an antibody library obtained in the step (iii) as panning pool. The present disclosure further provides the antibody panning pool obtained by this method. The panning pool may be used to further screen and obtain desired antibodies according to the needs.

In a specific embodiment, the mutant antigen is directly immobilized on the support, and in another specific embodiment, the mutant antigen is on a membrane with phospholipid membrane structure.

In a third aspect, the present disclosure provides a method for isolating antibodies in an antibody library, and the method includes the following steps:
(i) providing a support, herein the support is immobilized with mutant antigens with incorporation of amino acids with photo cross-linking activity or its derivative thereof in or near a target epitope of the target antigen;
(ii) providing conditions that enables the contact of the mutant antigen with antibody display library, and applying ultraviolet irradiation with suitable wavelength and energy to allow the said mutant antigen to be covalently cross-linked to displayed antibodies in the library that binds to or near the target epitope to form an antigen-antibody complex; and
(iii) performing elution under certain conditions, wherein this condition enables antibodies that are not covalently cross-linked to the mutant antigen to be washed away from the support, while the displayed antibodies that forms covalent crosslinking remains on the support, thereby antibodies binding to the target epitope are isolated form those that do not bind to the target epitope in the library.

The specific embodiments described above regarding the first aspect of the present disclosure may also be applied to the methods of the second and third aspects of the present disclosure.

In a specific embodiment, the mutant antigen is directly immobilized on the support. In another specific implementation solution, the mutant antigen is expressed on a membrane with a phospholipid structure.

In a fourth aspect, the present disclosure provides a method for selecting antibodies against a specific epitope of an antigen, and the method includes:
(i) mutant antigens incorporated with amino acids with photo cross-linking activity or its derivative in or near a target epitope is brought into contact with a labeled antibody display library under conditions that allows the antigen to bind to the antibody, and irradiated with suitable wavelength and energy to allow the mutant antigen to be covalently cross-linked to the displayed antibody that binds to or near the target epitope to form an antigen-antibody complex;
(ii) selecting the antigen-antibody complexes that form covalent crosslinking with the mutant antigen, and releasing the displayed antibodies that form the covalent crosslinking with the mutant antigen; and optionally
(iii) further selecting antibodies capable of binding to the target antigen from the displayed antibodies obtained in the step (ii).

In some embodiments, the mutant antigen makes contact with the antibody display library in solution. In some embodiments, the mutant antigen is expressed on the cell membrane. In some embodiments, cells expressing the mutant antigen are selected by flow cytometry. In some embodiments, the mutant antigen is a transmembrane protein.

In a fifth aspect, the present disclosure provides an antibody or an antigen-binding fragment thereof that specifically binds to complement C5a obtained by using the antibody selection method of the present disclosure. The said antibody or antigen-fragment binding to complement C5a comprises at least one immunoglobulin single variable domain, herein the at least one immunoglobulin single variable domain contains the following complementary determining regions (CDRs): HCDR1: SEQ ID NO. 23, HCDR2: SEQ ID NO. 24 and HCDR3: SEQ ID NO. 25; or a variant thereof. In some embodiments, the immunoglobulin single variable domain comprises a heavy chain variable region (VH) containing an amino acid sequence of SEQ ID NO. 29, or a variant thereof. In some embodiments, the immunoglobulin single variable domain comprising VH containing an amino acid sequence of SEQ ID NO. 31, or a variant thereof.

In some embodiments, the antibody or the antigen-binding fragment thereof that specifically binds to C5a further includes a second variable domain. In some embodiments, the second variable domain contains the following CDRs: LCDR1: SEQ ID NO. 26, LCDR2: SEQ ID NO. 27, and LCDR3: SEQ ID NO. 28; or a variant thereof; in some embodiments, the second variable domain is a light chain variable region (VL) containing an amino acid sequence of SEQ ID NO. 30; or a variant thereof.

In some embodiments, the antibody or the antigen-binding fragment thereof that specifically binds to complement C5a contains VH with the amino acid sequence shown in SEQ ID NO: 29 and VL with the amino acid sequence shown in SEQ ID NO: 30; or a variant thereof. In some embodiments, the antibody that specifically binds to complement C5a contains VH with the amino acid sequence shown in SEQ ID NO:31 and VL with the amino acid sequence shown in SEQ ID NO:30; or a variant thereof.

In some embodiments, the antibody or the antigen-binding fragment thereof that specifically binds to complement C5a further contains an immunoglobulin Fc region. In some embodiments, the Fc region is a human Fc. In some embodiments, the human Fc is an IgG1 Fc. In some embodiments, the antibody or the antigen fragment thereof that specifically binds to complement C5a is a heavy chain antibody. The heavy chain antibody contains a single variable domain of an immunoglobulin, and the single variable domain contains the following CDRs: HCDR1: SEQ ID NO. 23, HCDR2: SEQ ID NO. 24 and HCDR3: SEQ ID NO. 25; or a variant thereof. In some embodiments, the single variable domain is VH contains the amino acid sequence shown in SEQ ID NO. 29; or a variant thereof. In some embodiments, the single variable domain is VH contains the amino acid sequence shown in SEQ ID NO. 31; or a variant thereof. In a further embodiment, the heavy chain antibody is VHH, which consists of the single variable domain as described above.

In some embodiments, the aforementioned antibody or antigen-binding fragment thereof that specifically binds to C5a further contains a second variable domain. In some embodiments, the second variable domain contains the following CDRs: LCDR1: SEQ ID NO. 26, LCDR2: SEQ ID NO. 27 and LCDR3: SEQ ID NO. 28; or a variant thereof. In some embodiments, the second variable domain is a VL containing the amino acid sequence of SEQ ID NO. 30; or a variant thereof. In some embodiments, the antibody or the antigen-binding fragment thereof that specifically binds to C5a comprises a VH containing the amino acid sequence of SEQ ID NO:29 and a VL containing the amino acid sequence of SEQ ID NO:30; or a variant thereof. In some embodiments, the antibody or the antigen-binding fragment thereof that specifically binds to C5a comprises a VH containing the amino acid sequence of SEQ ID NO:31 and a VL containing the amino acid sequence of SEQ ID NO:30; or a variant thereof. In some embodiments, the antibody or the antigen-binding fragment thereof that specifically binds to C5a contains scfv containing a nucleic acid sequence shown in SEQ ID NO. 19 or the amino acid sequence shown in SEQ ID NO. 20; or a variant thereof. In some embodiments, the antibody or the antigen-binding fragment thereof that specifically binds to a complement C5a epitope comprises scfv containing a nucleic acid sequence shown in SEQ ID NO. 21 or an amino acid sequence shown in SEQ ID NO. 22; or a variant thereof. In some embodiments, the antibody or the antigen-binding fragment thereof that specifically binds to complement C5a of the present disclosure further comprises an immunoglobulin Fc region. In some embodiments, the Fc is a human Fc. In some embodiments, the human Fc is an IgG1 Fc. In some embodiments, the antibody or the antigen-binding fragment thereof that specifically binds to complement C5a has IgG structure, namely it comprises heavy chain and light chain.

The methods for selecting antibodies against epitopes of the present disclosure has a wide range of application: it is not only applicable to the soluble proteins, but also to the transmembrane proteins expressed on the phospholipid membrane structures; and the proteins or cells expressing the transmembrane proteins can be immobilized on a support for screening, and can also be carried out in a solution without a support.

### Brief Description of the Drawings

The drawings constituting the present application are used to provide a further understanding of the present disclosure. The exemplary embodiments and descriptions are used to explain the present disclosure, and do not constitute improper limitation of the present disclosure. In the drawings:
Fig. 1 shows the SDS-PAGE of wild-type (WT) IL1β and pBpa mutants.
Fig. 2 (Fig.2A and Fig.2B) shows the of enzyme-linked immunosorbent assay (ELISA) results of WT IL1β and pBpa mutants with Canakinumab (2A) or Gevokizumab (2B).
Fig. 3 shows the Western blot detection of WT IL1β and pBpa with Canakinumab or Gevokizumab, herein "-" represents no UV irradiation treatment; and "+" represents UV irradiation treatment.
Fig. 4 (Fig.4A and Fig.4B) shows the affinity detection of phage target with WT IL1β, 64pBpa(A) and 63pBpa(B), herein *p<0.05, **p<0.01, ***p<0.001, and ^{ns}p≥0.05.
Fig.5 (Fig.5A and Fig.5B) is the ELISA result of a monoclonal phage and IL1β with alanine mutation at different sites, herein p value is the comparison between the alanine mutant group and the wild-type group, *p<0.05, **p<0.01 ,***p<0.001, and ^{ns}p≥0.05.
Fig.6 (Fig.6A and Fig.6B) shows the affinity of phage and antigen, herein 6A is ELISA results of hC5a-35 phages with WT hC5a and 18pBpa; 6B is ELISA results of E02 phages with WT hC5a and alanine mutant, *p<0.05.
Fig. 7 (Fig.7A and Fig.7B) shows the affinity detection of hC5a-35-E02 phage with hC5a(7A) and mC5a(7B), *p<0.05, and ns means no statistical difference.
Fig. 8 (Fig.8A and Fig.8B) shows the affinity of hC5a-35 phage with WT hC5a, and hC5a mutants in which pBpa is incorporated at different positions.
Fig. 9 shows the Western Blotting detection of E02-scFv-Fc fusion protein binding to 18pBpa.
Fig. 10 shows the ELISA results of #8, #62, #125, #137 monoclonal phage libraries with BSA, WT GFP, and pBpa GFP.
Fig. 11 shows the expression of A2A and pBpa-A2A on the surface of Hela cells detected by flow cytometry.

### Detailed Description of the Embodiments

The present disclosure is described in detail herein by using the following definitions and reference to the examples.

As used herein, the term "amino acid with photo cross-linking activity" refers to an amino acid that can covalently crosslink with an amino acid residue of adjacent proteins under suitable light irradiation conditions. The "amino acid with photo cross-linking activity" may include natural amino acids or ncAAs. The "photo cross-linking activity" includes, but is not limited to, sensitivity to ultraviolet (UV) light. Non-limiting examples of the "amino acid with photo cross-linking activity" include pBpa, pAzF and the like.

As used herein, the term "noncanonical amino acid" refers to an amino acid that is not one of 20 classic amino acids or selenocysteine or pyrrolysine. Other terms that can be used synonymously with the term "noncanonical amino acid" are a "non-naturally encoded amino acid", an "unnatural amino acid", and a "non-naturally occurring amino acid". The term "noncanonical amino acid" also includes, but is not limited to, amino acids that are modified (e.g., post-translational modification) by naturally-encoded amino acids (including but not limited to 20 common amino acids or pyrrolysine and selenocysteine), but they themselves are not naturally incorporated into the growing polypeptide chain through the translation complex. The "noncanonical amino acid" may include a variety of functional groups or active groups, which can provide additional functions and/or activities.

As used herein, the term "photo cross-linking" means that under the suitable light irradiation condition, the groups of amino acids with photo cross-linking activity will covalently cross-link with groups of amino acid residues of adjacent proteins to form complex.

As used herein, "antibody display carrier" or "antibody library display carrier" is not limited to a particular vector. Although the present disclosure is exemplified with reference to phage display, the "antibody display library" of the present disclosure can also be identified by other display and enrichment techniques. Antibody fragments have been displayed on the surface of filamentous bacteriophage encoding antibody genes (Hoogenboom and Winter, J Mol Biol, 222:381-388 (1992); McCafferty et al., Nature 348(6301):552-554 (1990); Griffiths et al. EMBO J, 13(14):3245-3260(1994)). For a review of techniques for selecting and screening antibody display libraries, referring to, for example, Hoogenboom, Nature Biotechnol. 23(9): 1105-1116 (2005). In addition, it is known in the art to display heterologous proteins and its fragment on the surface of Escherichia coli (Agterberg et al., Gene 88:37-45 (1990); Charbit et al., Gene 70:181-189 (1988); Francisco et al., Proc. Natl. Acad. Sci. USA 89: 2713-2717 (1992)), and yeast such as Saccharomyces cerevisiae (Boder and Wittrup, Nat. Biotechnol. 15: 553-557 (1997); Kieke et al., Protein Eng. 10: 1303-1310 (1997)). Other known display techniques include ribosome or mRNA display (Mattheakis et al., Proc. Natl. Acad. Sci. USA 91:9022-9026 (1994); Hanes and Pluckthun, Proc. Natl. Acad. Sci. USA 94:4937 -4942 (1997)), DNA display (Yonezawa et al., Nucl. Acid Res. 31(19):e118 (2003)); microbial cell display such as bacterial display (Georgiou et al., Nature Biotech. 15:29-34 (1997)), display on mammalian cells, spore display (Isticato et al, J. Bacteriol. 183: 6294-6301 (2001); Cheng et al, Appl. Environ. Microbiol. 71: 3337-3341 (2005) and US 60/865,574 filed on November 13, 2006), viral display such as retroviral display (Urban et al, NucleicAcids Res. 33:e35 (2005), protein-DNA ligation based display (Odegrip et al, Proc. Acad. Natl. Sci. USA 101:2806-2810 (2004)); Reiersen et al, Nucleic Acids Res. 33:e10 (2005)), and microbead display (Sepp et al, FEBS Lett. 532:455-458 (2002)).

The "antibody phage display library" refers to a phage display library that displays antibodies or antibody fragments. The library can be monovalent, displaying one single-chain antibody or antibody fragment on average per phage particle, or multivalent, displaying an average of two or more antibodies or antibody fragments per viral particle. The term "antibody fragment" includes, but is not limited to, single-chain Fv (scFv) fragments and Fab fragments.

The "displayed antibodies" refers to antibodies or antibody fragments that are displayed on the surface of library carrier (for example, phages, bacteria, yeast, or mammalian cells) and can be exposed to the antigen used for screening. Thus, in the present disclosure, when referring to the "displayed antibodies", it generally refers to the antibody together with the carrier on which it is displayed, unless otherwise understood by those skilled in the art according to the context. For example, when the phage display library is used, eluting the "displayed antibodies" from the support means eluting the phage displaying the corresponding antibody from the support.

The "display library" refers to the general term for the population of molecules presented on the display carrier. For antibody display libraries, the population includes, but is not limited to, antibodies, antibody fragments, nanobodies, scFv, Fab, VH, VL, protein/antibody fusion molecules, and the like.

### Effect of present disclosure

Compared with conventional affinity-based antibody screening and separating methods, the method of the present disclosure can minimize the enrichment/screening bias due to the differences in the initial affinity of different antibodies in the library for the target antigen, and improve the diversity of the antibody pool during the selecting process.

### Embodiment

It should be noted that, in the case no conflict, the embodiments in the present application are merely illustrative, and are not intended to limit the present disclosure in any manner.

### Embodiment 1: IL1β incorporated with pBpa on epitope capable of crosslinking with antibody

### 1. Selection of epitope

IL1β is an important cytokine that mediates inflammatory responses and various physiological activities. Canakinumab is a monoclonal antibody that blocks the interaction of human IL1β with IL1 receptor, and was approved by Food and Drug Administration (FDA) for clinical use. On the basis of the crystal structure of the Canakinumab-IL1β complex (Protein Data Bank (PDB) database ID number: 4G6J), the antigen epitope that Canakinumab binds includes Ser21, Glu25, Lys27, Glu64, Lys65, Asn66 and Asn129 (M. Blech et al, Journal of molecular biology 2013, 425:94). Among these, residues Glu64, Lys65, and Asn66 residues of IL1β are in a flexible loop and form extensive interactions with CDR3 of VH and CDR1 and CDR3 of VL. Another neutralizing antibody, Gevokizumab (PDB database ID number: 4G6M), not only binds to a distinct epitope, but also blocks the interaction between IL1β and IL1R, IL1 receptor accessory protein (IL1RAcP) (M. Blech et al, Journal of molecular biology 2013, 94:425; D. Wang et al., Nature immunology 2010, 11(10):905-911). IL1β residues 63-66 are selected as the target epitope to develop and validate the new selection method. Residues Ala2, Leu7 of the antigen are greater that 15Å away from loop 63-66 and does not form any direct interactions with Canakinumab or Gevokizumab, and are therefore selected as negative controls of the undesired binding site.

### 2. Incorporation of pBpa into IL1β

### 2.1 Construction of wild-type(WT) hIL1β and its mutants

There are numerous previous reports that the *Methanocaldococcus jannaschii* (Mj) tyrosine tRNA synthetase (MjTyrRS) mutant and MjtRNA_{CUA} pair w can be efficiently incorporated in the wild-type proteins through genetic codon expansion method (Wang L et al. Chem Biol 2001, 8(9): 883-90; Jiantao Guo et al. Angew Chem Int Ed Engl 2009, 48(48): 9148-9151; Wang L et al. Science 2001, 292(5516): 498-500; Chin JW. Nature 2017, 550(7674): 53-60]. pBpa may also function efficiently in this system and be incorporated into proteins (Hino N, et al. Nat Methods. 2005; 2(3): 201-6. Dormán G et al. Chem Rev 2016, 116(24): 15284-15398; Joiner MC et al, Protein Sci. 2019 Jun; 28(6): 1163-1170). In this example, recombinant plasmids expressing IL1β wild-type or mutant with the succinate codon TAG incorporation at 63, 64, 65, 66, 2 or 7 (designated as 63pBpa, 64pBpa, 65pBpa, 66pBpa, 2bpa or 7pBpa) is constructed.

In this example, all plasmids were generated by the Gibson assembly method (Daniel G. Gibson et al. Nucleic Acids Res 2009, 37(20):6984-6990). The open reading frames of IL1β fused with a 6×Histidine at the C terminus were amplified from pUC57-IL1β with primers IL1β-WT-F/R (see Table 1), and inserted into a linearized pET28a vector (Novagen, 69864-3) vector (digested with Nco I and *Nhe* I). Site-directed mutagenesis was performed to generate plasmids pET28a-IL1β-2TAG, pET28a-IL1β-7TAG, pET28a-IL1β-63TAG, pET28a-IL1β-64TAG, pET28a-IL1β-65TAG, and pET28a-IL1β-66TAG using according to the manufacturer's instructions (Yeasen Biotech, 10911) by using method of seamless and rapid cloning. pBpa was incorporated into IL-1β of these plasmids (J&K Scientific, 204322, CAS104504-45-2) through genetic codon expansion method. The same method was applied to the construction of pET28a-IL1β-2Ala, pET28a-IL1β-7Ala, pET28a-IL1β-63Ala, pET28a-IL1β-64Ala, pET28a-IL1β-65Ala, pET28a-IL1β-66Ala and pET28a-IL1β-63-66Ala, which was used to express alanine mutants of IL1β.

**Table 1: Primers and gene sequences**

| Primers | Sequences |
|---|---|
| pBpa mutant primers | |
| 2TAG-F | TAAGAAGGAGATATACCATGTAGCCTGTGCGGAGCCT |
| 7TAG-F: | TAAGAAGGAGATATACCATGGCGCCTGTGCGGTAGCTGAACTGTACC |
| 63TAG-F | CATTGGGCCTTTAGGAAAAGAATC |
| 63TAG-R | GATTCTTTTCCTAAAGGCCCAATG |
| 64TAG-F | TGGGCCTTAAGTAGAAGAATCTGT |
| 64TAG-R | ACAGATTCTTCTACTTAAGGCCCA |
| 65TAG-F | GCCTTAAGGAATAGAATCTGTACC |
| 65TAG-R | GGTACAGATTCTATTCCTTAAGGC |
| 66TAG-F | TTAAGGAAAAGTAGCTGTACCTGA |
| 66TAG-R | TCAGGTACAGCTACTTTTCCTTAA |

| Alanine mutant primers | |
|---|---|
| K63A-F | CATTGGGCCTTGCGGAAAAGAATC |
| K63A-R | GATTCTTTTCCGCAAGGCCCAATG |
| E64A-F | TGGGCCTTAAGGCAAAGAATCTGT |
| E64A-R | ACAGATTCTTTGCCTTAAGGCCCA |
| K65A-F | GCCTTAAGGAAGCGAATCTGTACC |
| K65A-R | GGTACAGATTCGCTTCCTTAAGGC |
| N66A-F | TTAAGGAAAAGGCTCTGTACCTGA |
| N66A-R | TCAGGTACAGAGCCTTTTCCTTAA |
| K63AN66A-F | CATTGGGCCTTGCGGCAGCGGCTCTGTACCTGAG |
| K63AN66A-R | CTCAGGTACAGAGCCGCTGCCGCAAGGCCCAATG |

| scFv phage construction primers | |
|---|---|
| Canakinum ab-scFv-F | GGCCCAGGCGGCCGAGATTGTCCTTACCCAGAGTCC |
| Canakinum ab-scFv-R | GGCCGGCCTGGCCACTAGTAAGGGTTGGGGCGGATGCACTCCCACTGCTG ACGGTTACC |
| Gevokizuma b-scFv-F | GGCCCAGGCGGCCGACATACAGATGACCCAATCCAC |
| Gevokizuma b-scFv-R | GGCCGGCCTGGCCACTAGTAAGGGTTGGGGCGGATGCACTCCCGGATGAG ACCGTCACG |
| 64UV63-scFv-Fc-F | AATTCGGCGGCCCAGGCGGCCGAGCTCACACTCACGCAGTCT |
| 64UV63-scFv-Fc-R | AGATGCCAGGCCGGCCTGGCCACTAGTGAGGGTTGGGGCGGA |

| pBpa mutant construction primers | |
|---|---|
| hC5a-18TAG-F | ATATAAACATTCAGTATAGAAGAAATGTTGTTACGATG |
| hC5a-18TAG-R | CATCGTAACAACATTTCTTCTATACTGAATGTTTATAT |

| Alanine mutant construction primers | |
|---|---|
| V18A-F | ACGCTGCAAAAGAAGATAGAAGAAATAGCTGCTAAATATAAACATTCAGTAGC GAAGAAATG |
| V19A-F | ACGCTGCAAAAGAAGATAGAAGAAATAGCTGCTAAATATAAACATTCAGTAGTG GCGAAATG |
| V18A-K19A-F | ACGCTGCAAAAGAAGATAGAAGAAATAGCTGCTAAATATAAACATTCAGTAGC GGCGAAATG |

**Table 2: Sequence table**

| Gene | Nucleic acid sequence (SEQ ID NO.) | Amino acid sequence (SEQ ID NO.)) |
|---|---|---|
| IL 1β WT with 6xHis tag | 1 | 2 |
| Canakinumab HC | 3 | 4 |
| Canakinumab LC | 5 | 6 |
| Gevokizumab HC | 7 | 8 |
| Gevokizumab LC | 9 | 10 |
| Canakinumab scFv | 11 | 12 |
| Gevokizumab scFv | 13 | 14 |
| 64UV63 scFv | 15 | 16 |
| hC5a with 6×His tag | 17 | 18 |
| hC5a-35 scfv | 19 | 20 |
| E02-scfv | 21 | 22 |
| HCDR1 | | 23 |
| HCDR2 | | 24 |
| HCDR3 | | 25 |
| LCDR1 | | 26 |
| LCDR2 | | 27 |
| LCDR3 | | 28 |
| hC5a-35 VH | | 29 |
| hC5a-35 VL | | 30 |
| E02 VH | | 31 |

### 2.2 Expression of WT hIL1β and its mutants

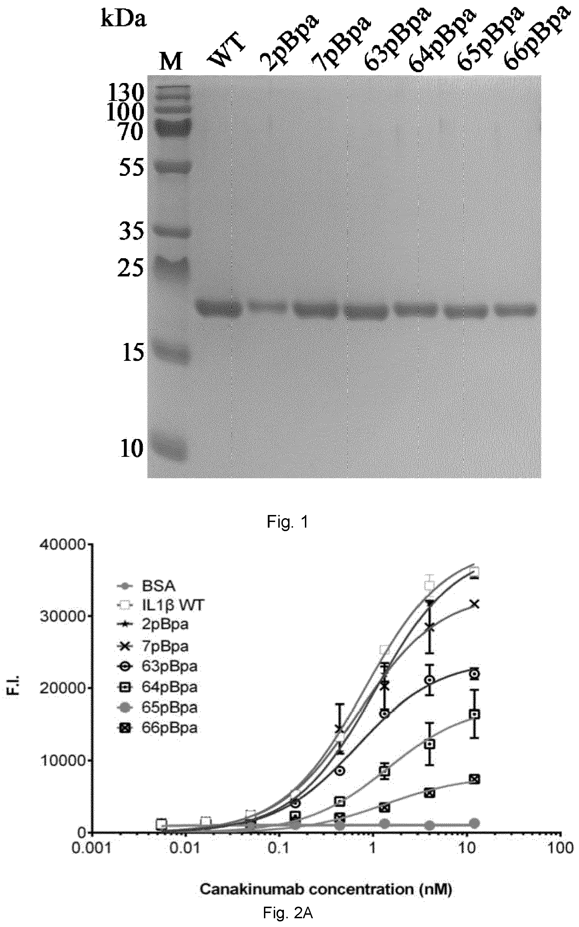

Plasmid encoding the IL1β mutant and pEVOL-pBpaRS vector (Young TS et al. Mol Biol 2010, 395(2):361-74) (MjTyrRS-tRNA_{CUA} pair containing Y32G, V103L, E107P, D158T and I159S mutations) were co-transformed into Escherichia coli BL21 (DE3). Only cells containing the double plasmids expressed the full-length protein in the presence of pBpa, which were purified by Ni-NTA column chromatography followed by size exclusion chromatography (SEC). The cells were cultured to OD600=0.6 in 2×YT medium, then, 1 mM pBpa, 0.5 mM isopropyl-β-d-thiogalactoside (IPTG) and 0.2% arabinose were added, and cultured overnight at 37°C. The yield of these mutant proteins was from 8 to 43 mg/L. Proteins were analyzed by SDS-PAGE (Fig. 1) and electrospray ionization mass spectrometry (ESI-MS) (data was not shown) to confirm the incorporation of pBpa. IL1β wild-type and mutant proteins migrated as a single band at approximately 19 kDa on SDS-PAGE gel, and exhibited the expected mass that was consistent with its amino acid sequence.

### 3. Binding ability of WT IL1β and its mutants to antibody

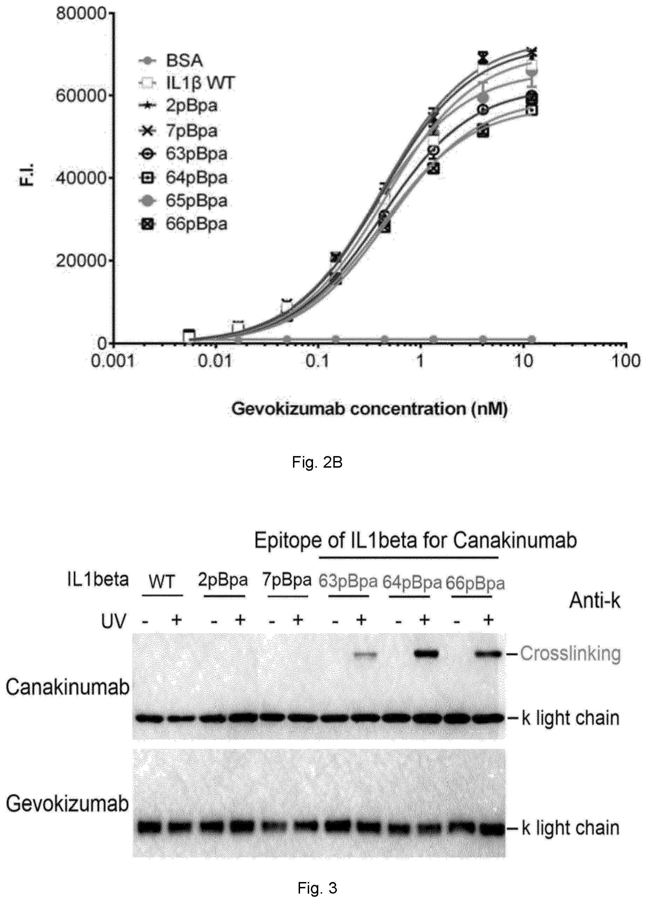

ELISA results show that compared with the binding of wild-type IL1β to Canakinumab, the affinity of the IL1β mutant to Canakinumab was reduced, and it was concentration-dependent (Fig. 2A). In contrast, the binding of Gevokizumab to wild-type IL1β and mutant IL1β was not significantly different (Fig. 2B), so Gevokizumab was used as a negative antibody control in the following experiments.

### 4. Verification of photo cross-linking between IL1β with incorporation of pBpa on epitope and antibody

IL1β WT and mutants (0.5 mg/ml) were incubated with Canakinumab (1 mg/ml), respectively, and exposed to long UV irradiation (6 W, and 365 nm) for 10 hours according to the method used in other protein cross-linking researches (Sato S et al. al, Biochemistry. 2011;50(2):250-7. Results of Western Blot (Fig. 3) showed that mutant strains 63pBpa, 64pBpa and 66pBpa form covalently crosslinked products with light chain of Canakinumab, while 2pBpa and 7pBpa did not form the covalently crosslinked products. Mass spectrometry data also supported these results (not shown), confirming that pBpa in IL1β can cross-link with adjacent binding antibodies under UV irradiation, in contrast, no cross-linking of Gevokizumab (the binding of Gevokizumab to IL1β was away from loop 63-66 or loop 2-7 of IL1β, and the distance between the two proteins was greater than 13Å) was observed under the same conditions.

The results above showed that: when pBpa was incorporated into the epitope and its vicinity, the spatial distance of photo cross-linking is exactly the same as the spatial distance of antigen-antibody binding. Under UV irradiation, the antibody could covalently cross-link with pBpa-incorporated antigen to form new complex, while non-epitope with too large spatial distance will not undergo photo cross-linking. The pBpa-incorporated IL1β on the epitope can undergo photo cross-linking reaction with antibodies, laying the foundation for screening epitope-directed antibodies by specific photo cross-linking.

### Embodiment 2

### Epitope-directed screening of fully human antibody phage library

### 1. Construction of a fully human antibody phage library

According to published methods (Barbas CF et al, Proc Natl Acad Sci USA 1991, 88(18):7978-7982; Barbas III CF, Dennis RB, Gregg JS, In Phage Display: A Laboratory Manual. (CSH Press, 2001)), a multivalent single-chain antibody plll phage display library was previously constructed by using B cells from human peripheral blood mononuclear cells (PBMC), and the library had an antibody sequence diversity of about 10⁹ cfu.

### 2. Screening of fully human antibody phage library by specific photo cross-linking

The ELISA plate (Corning Costar, 2592) was coated with 100 µl 0.1 mg/ml mutant proteins (diluted with Dulbeccos Phosphate-Buffered Saline (DPBS)) and incubated overnight at 4°C. On the next day, the coating solution was removed, and 200 µl of blocking solution (3% skimmed milk, DPBST, 0.25% Tween 20) was used for blocking at 37°C for 2 h. After removing the blocking solution, 10¹⁰ pfu of phage was added and incubated with the mutant proteins 63pBpa and 64pBpa for a period of time, and then UV irradiation (6 W, and 365 nm) was applied for 15 min-2 h. After washing with routine washing solution, three rounds of competitive washing (DPBS, 0.25% Tween 20, pH 7.4, and 0.1 mg/ml IL1β WT) was performed. After washing with routine washing solution, three rounds of low-pH washing (300 mM NaCl, 3% Tween 20, 100 mM glycine, and pH 2.0) was performed to remove non-covalently bound phage, followed by three rounds of PBS washing. After washing steps, the covalently cross-linked phage-antigen complex was released from the well by trypsin digestion. The collected phases from each well were incubated with *E.coli* XL1-Blue strain to infect cells. Colony Forming Unit (CFU) was counted, and finally the positive clones(hits) were picked. As expected, the output CFUs from panning of the both mutants were very low. Nonetheless, the output CFU is 3-4-fold higher compared to the group without UV irradiation (designated as non-UV-treated group), suggesting that a substantial portion of the output phage pool was covalently cross-linked with 63pBpa or 64pBpa (Table 3). In contrast, panning against WT IL1β using the same phage library and the same method exhibited a output UV/non-UV output ratio of 1.2 (close to 1), indicating that no significant cross-linking happened without incorporated pBpa. In addition, monoclonal phages displaying the scFv of Canakinumab and Gevokizumab were generated and selected following the same protocol, respectively. The UV/non-UV output ratio of the Canakinumab-scFV phages was 3.8. In contrast, the negative control antibody Gevokizumab-scFv phages exhibited a ratio of 1.1, indicating no significant number of the phage cross-linked with IL1β.

**Table 3: Output CFU ratios of different phage libraries for WT IL1β and pBpa mutants with or without UV treatment**

| Sample | Screened antigen | UV-treated group output CFU | Non-UV-treated group output CFU | UV-treated output CFU / Non-UV-treated output CFU |
|---|---|---|---|---|
| Fully human antibody phage library | WT L1β | 776 | 648 | 1.2 |
| | 64pBpa | 268 | 86 | 3.1 |
| | 63pBpa | 981 | 261 | 3.7 |
| Canakinumab scFv phage | 63pBpa | 6400 | 1696 | 3.8 |
| Gevokizumab scFv phage | 63pBpa | 8650 | 7900 | 1.1 |

### 3. Selection and analysis of clones

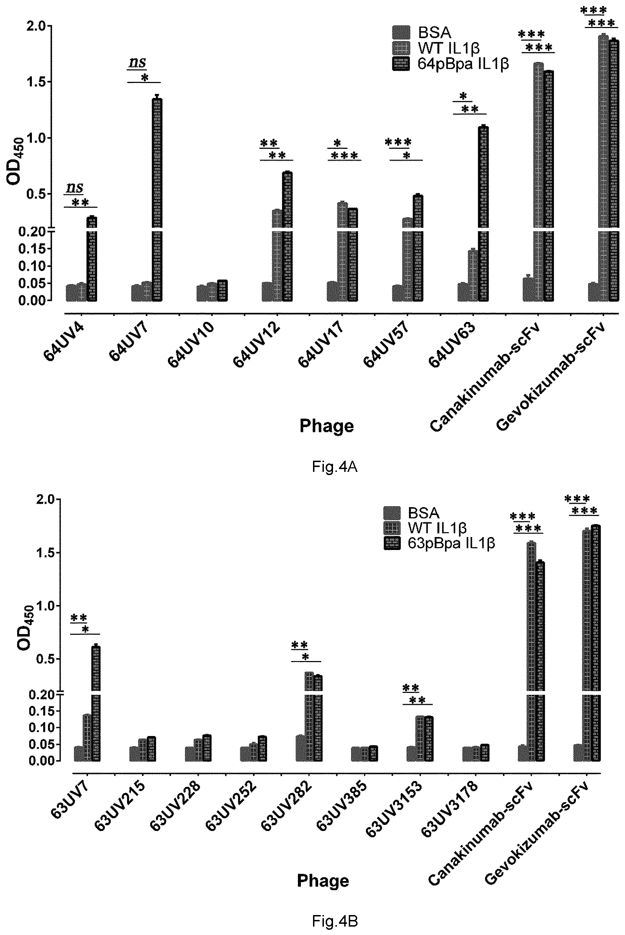

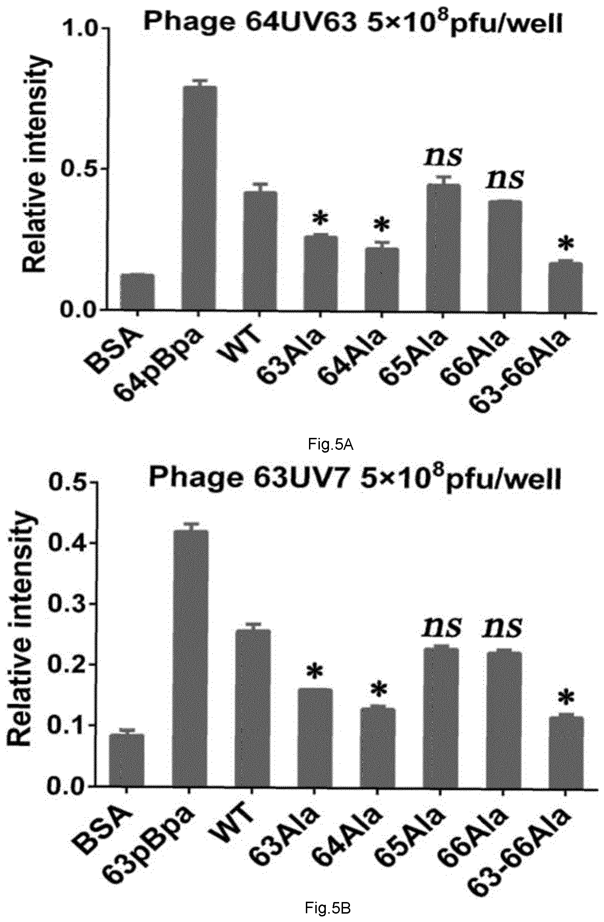

55 colonies from the hit pool of 63pBpa or 64pBpa were randomly picked and their sequences were analyzed. Results showed that the sequences are diverse with low homology. In these, 15 (7 and 8 from the hit pool of 63pBpa and 64pBpa, respectively) distinct sequences were selected to generate monoclonal phages. The binding affinities of these phages to for WT IL1β and mutant 63pBpa and 64pBpa were analyzed by the ELISA. As shown in Figs. 4A and 4B, more than half of phages selected from selection on 63pBpa or 64pBpa were cross-reactive with WT IL1β wild-type, although some of them showed reduced affinities. Two monoclonal phages (designated as 63UV7 and 64UV63 respectively) with significant affinities to both WT-IL1β and 63pBpa or 64pBpa, respectively, were picked and then incubated with 63pBpa or 64pBpa and processed with the panning procedure. After elution, the output CFUs of UV and non-UV-treated groups were counted and compared. The CFU of UV-treated group was 4-6-fold higher than that of the non-UV-treated group (Table 4), demonstrating that these scFv-displaying monoclonal phages bind to the desired epitope, and could cross-link with 64pBpa or 63pBpa upon UV irradiation. As a control, these phages did not show much difference on the CFUs between UV- and non-UV-treated groups against WY IL1β (Table 4). In addition, Lys63Ala, Glu64Ala, Lys65Ala, and Asn66Ala single mutants and Lys63Ala-Asn66Ala quadruple mutant of IL1β were also generated. Phages 64UV63 and 63UV7 showed significantly lower affinities to some of these alanine mutants compared to the WT, 63pBpa or 64pBpa (Fig. 5), indicating that they bind to the target epitope.

In conclusion, it is feasible and efficient to screen epitope-directed antibodies by the fully human antibody phage library in the specific photo cross-linking method.

**Table 4: Output CFU ratios of targets selected from fully human antibody phage library against WT IL1β, 63pBpa and 64pBpa with or without UV treatment**

| Sample | Screened antigen | UV-treated group output CFU | Non-UV-treated group output CFU | UV-treated output CFU / Non-UV-treated output CFU |
|---|---|---|---|---|
| 63UV7 phage | WTIL1β | 184 | 197 | 0.9 |
| | 63pBpa | 201 | 46 | 4.4 |
| 64UV63 phage | WT IL1β | 128 | 117 | 1.1 |
| | 64pBpa | 140 | 22 | 6.4 |
| Canakizumab scFv phage | 63pBpa | 7900 | 2120 | 3.7 |
| Gevokizumab scFv phage | 63pBpa | 4000 | 3040 | 1.3 |

### Embodiment 3

### Epitope-directed selecting of mouse immune antibody phage library (also applicable to humanized antibody transgenic mice)

### 1. Screening of mouse immune antibody phage library by specific photo cross-linking

A large number of researches show that mouse immunization is a popular approach to generate antibodies with high affinity and selectivity against an antigen. The epitope-directed antibody selection method to the phage library produced from mouse immunization approaches. Mice were routinely immunized for three times with WT IL1β. Once the antibody titer in serum was confirmed, their spleens were collected and used to generate phage display libraries (Barbas CF et al, Proc Natl Acad Sci USA 1991, 88(18):7978-7982; Barbas III CF, Dennis RB, Gregg JS, In Phage Display: A Laboratory Manual. (CSH Press, 2001)). These libraries were then applied to epitope-directed panning using a similar method described in Embodiment 2, except that two rounds of panning were applied against 64pBpa to further enrich the hits. The output CFUs from the UV-treated group were about 9 times higher than those of the non-UV-treated group. In contrast, the UV/non-UV ratios of the hit pool and the selected hits were all around 1 when WT IL1β was used as the antigen (Table 5).

**Table 5: Output CFU ratios of monoclonal phages targeting 64pBpa and derived from mouse immune phage libraries against 64pBpa, wild-type IL1β under UV or non-UV treatment**

| Sample | Coated antigen | UV-treated output CFU | Non-UV-treated output CFU | UV-treated output CFU / Non-UV-treated output CFU |
|---|---|---|---|---|
| Fully human antibody phage | 64pBpa | 3500 | 380 | 9.2 |
| | WT IL1β | 476 | 432 | 1.1 |
| i64UV9 phage | 64pBpa | 7500 | 1400 | 5.4 |
| | WT IL1β | 1400 | 1500 | 0.9 |
| i64UV120 phage | 64pBpa | 6600 | 1900 | 3.5 |
| | WT IL1β | 2500 | 2300 | 1.1 |
| i64UV5 phage | 64pBpa | 296 | 312 | 0.9 |
| | WT IL1β | 453 | 402 | 1.1 |
| i64UV40 phage | 64pBpa | 7900 | 7400 | 1.1 |
| | WT IL1β | 8300 | 8000 | 1.0 |
| i64UV104 phage | 64pBpa | 1300 | 980 | 1.3 |
| | WT IL1β | 1600 | 1200 | 1.3 |

| | | | | |
|---|---|---|---|---|
| Note: i represents that the phage was derived from the immunized mouse phage library | | | | |

### 2. Selection and analysis of clones

47 colonies from the hit pool were randomly picked and their sequences were analyzed. 7 sequence families were identified based on the homology. One representative clone was selected from each family, and generated monoclonal phages (except for one clone that yielded very low phage titer after production). Then, the output CFUs of these selected phages after one round of panning with or without the UV irradiation were examined. As shown in Table 2, 2 of the 6 selected monoclonal phages exhibited a UV/non-UV ratio larger than 3 against 64 pBpa, indicating their ability to cross-link with the target epitope. As a control, these phages did not show significant difference on the CFUs between UV and non-UV-treated groups against WT IL1β (Table 5).

In conclusion, it is feasible and efficient to select the epitope-directed antibodies by the mouse (also applicable to humanized antibody transgenic mice) immune antibody phage library through the specific photo cross-linking method.

### Embodiment 4

### Epitope-directed selecting of antibodies specific to human complement 5a (hC5a)

In order to show the general applicability of this method and prove the versatility of this method on other therapeutic targets, it is applied to antibody screening against hC5a antigen. Astherapeutic antibodies often require binding to a specific epitope in an antigen to exert their functions, this method could potentially facilitate antibody drug development (Daniel Ricklin et al. Nat Immunol 2010, 11(9):785-797; Cook WJ et al. Acta Crystallographica 2010, 66: 190-197; Toth MJ et al. Protein Sci 1994, 3(8): 1159-68). hC5a is a potential target for treatment of various diseases and syndromes such as anti-neutrophil cytoplasmic antibody-associated vasculitis (ANCA), atypical hemolytic uremic syndrome, systemic lupus erythematous, rheumatoid arthritis, and ischemia/reperfusion injury (Morgan BP et al. Nat Rev Drug Discov 2015, 14(12):857-77). In order to develop a therapeutic antibody against hC5a, it is desirable not only efficiently block the binding of hC5a to a hC5a receptor (C5aR), but also be highly selective to hC5a versus human C5 (hC5). By analyzing the crystal structures of hC5a (PDB: 3HQA) versus hC5 (PDB: 3CU7), an epitope (Ser16, Val17, Val18, Lys19 and Lys20), which is involved for the interaction with hC5a receptor (Huber-Lang MS, et al. J Immunol 2003, 170(12): 6115-24; Colley CS et al. MAbs 2018, 10(1): 104-117), but is buried inside the surface of hC5, was identified. Therefore, antibodies that bind to this epitope of hC5a are less likely to bind to hC5. Furthermore, the sequence of this epitope is highly conserved among human, monkey and rodent, which indicates that antibodies binding to this epitope are very likely cross-reactive among species.

In theme present disclosure, a Val18pBpa mutant of hC5a (designated as 18pBpa) was generated, characterized, and used for epitope-directed antibody selection. Panning was performed against the fully human phage displayed antibody library. After two rounds of screening against 18pBpa according to the selection procedure described above, the output ratio of UV/non-UV was greater than 13 (Table 6), suggesting that a significant portion of the output phage pool was covalently cross-linked with the antigen.

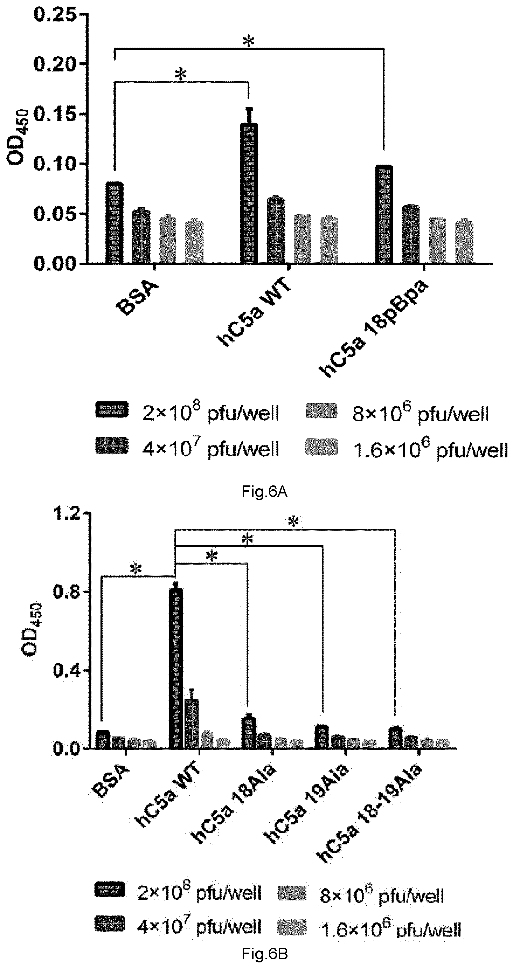

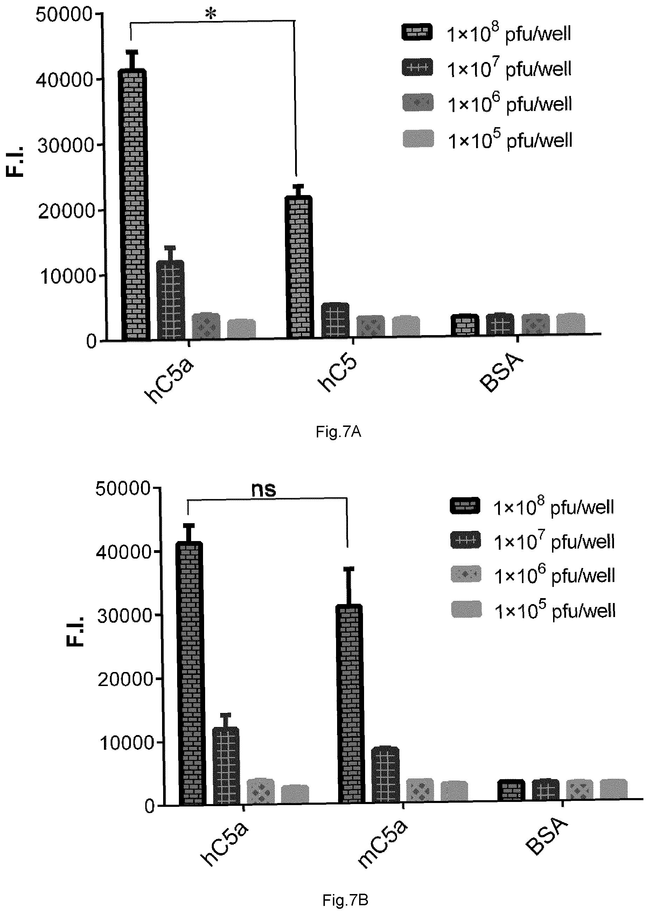

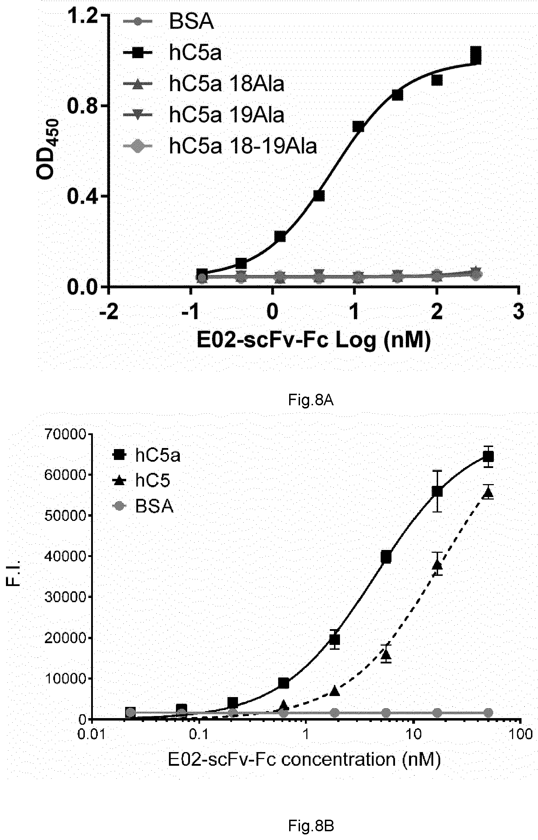

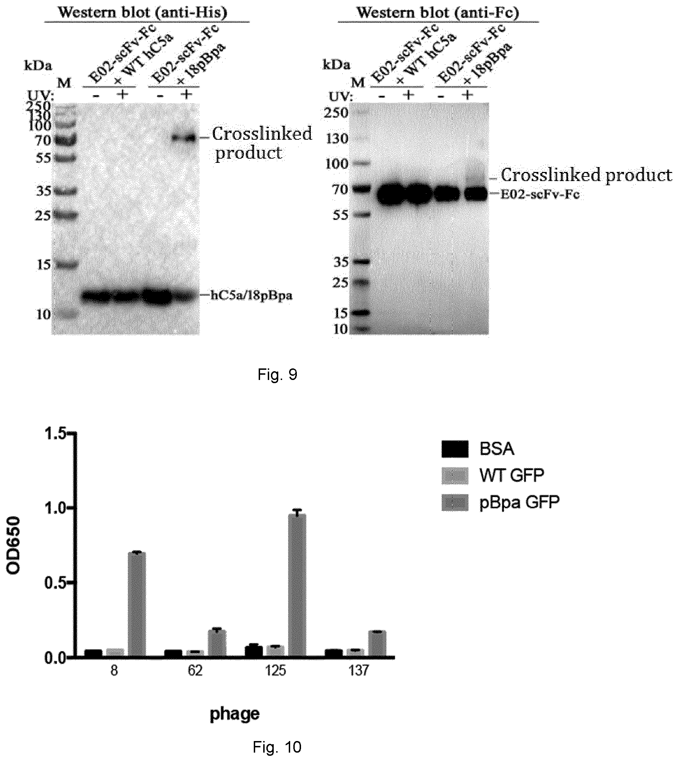

25 colonies from the hit pool are selected and their sequences were analyzed. Sequences with correct scFv sequence assemblies are clustered on the basis of homology. Hit hC5a-35scfv (SEQ ID NO.) was selected from the cluster with the most abundant homologous sequences. Although it only showed modest affinities to hC5a and low affinity to18pBpa (Fig. 6A), its UV/non-UV output CFU ratio was larger than 3 (Table 6). After affinity maturation on WT-hC5a using a secondary phage displayed antibody library generated by random mutagenesis based on the hC5a-35scfv sequence, a strong binder hC5a-35-E02 phage (E02) with the high affinity to hC5a was identified, and its UV/non-UV output ratio was significantly increased to 8.6 (Table 7). In order to verify whether this clone is a positive clone for the antigen epitope, it is verified by alanine scanning. Val18Ala and Lys19Ala single mutants and Val18Ala-Lys19Ala double mutants of hC5a were expressed and purified. Compared to WT-hC5a, E02 showed significantly lowered affinities for these alanine mutants, indicating that it binds to the target epitope (Fig. 6B). Furthermore, as expected, E02 showed much lower affinity to hC5 than hC5a (Fig. 7A), but similar affinity to mC5a (Fig. 7B). Next, E02 scFV-Fc fusion protein E02-scFv-Fc were also expressed and purified, and its binding affinities to hC5a, Val18Ala, Lys19Ala and Val18Ala-Lys19Ala, hC5 and mC5a were measured. The binding affinity profile corresponds to the results of E02 phages (Figs. 8A and 8B). Western blot results also showed that 18pBpa formed a covalently linker product with E02-scFv-Fc fusion protein, while WT-hC5a did not (Fig. 9).

In conclusion, the selection method for epitope-directed antibodies using specific photo cross-linking method has broad application value and universality.

**Table 6: Output CFU ratios of fully human antibody phage antibody library against WT hC5a, 18pBpa mutant with or without UV treatment**

| Sample | Screened antigen | UV-treated output CFU | Non-UV-treated output CFU | UV-treated output CFU / Non-UV-treated output CFU |
|---|---|---|---|---|
| Fully human phage antibody library | 18pBpa | 370 | 28 | 13.2 |
| | WT hC5a | 42 | 34 | 1.2 |

**Table 7: Output CFU ratio of monoclonal phage against wild WT hC5a, 18pBpa mutant under UV or non-UV treatment**

| Sample | Coated antigen | UV-treated output CFU | Non-UV-treated output CFU | UV-treated output CFU / Non-UV-treated output CFU |
|---|---|---|---|---|
| hC5a-35 phage | 18pBpa | 296 | 89 | 3.3 |
| | WT hC5a | 116 | 103 | 1.1 |
| E02 phage | 18pBpa | 670 | 78 | 8.6 |
| | WT hC5a | 101 | 97 | 1.0 |

### Embodiment 5: Epitope-directed selecting of antibodies against membrane surface protein

In order to prove the applicability of this method for screening antibodies against transmembrane proteins, we first applied to screen antibodies against antigens expressed on cell membranes. pCDNA3.1-WT GFP-GPI and pCDNA3.1-(TAC151TAG)GFP-GPI eukaryotic expression plasmids were constructed and transfected into 293T cells, respectively. After 48 hours, the results of flow cytometry showed that GFP-GPI was expressed on the membrane (data not provided). Then, the pcDNA3.1-(TAC151TAG)GFP eukaryotic expression plasmid was transfected into Hela cells, and 48 hours after transfection, the Hela cells were blocked with 5% milk for 1 hour, and incubated with a human natural antibody phage library (the titer is 10¹²/mL) on ice for 30 min. 365 nm UV irradiation was applied for 30min, elution with acidic eluent for 30 min was applied to remove non-covalently cross-linked phages The covalently cross-linker phages were released by 200 ug/mL trypsin at 37°C for 30 min and subjected to infect XL-blue for 1h, followed by counting of the number of single clones. The output ratio of UV/non-UV was 5.8 (see Table 8), indicating that the cross-linked phages generated by UV irradiation were enriched. Single colonies in the UV-treated group were amplified with pSEX-F and pSEX-R as upstream and downstream primers respectively, and colonies PCR products larger than 750 bp were subjected to phage packaging (low titer). At the same time, soluble WT GFP and pBpa-GFP were also expressed and purified in 293F cells. After coated in 96-well ELISA plate, and blocked with 5% milk for 1h at a room temperature, then phage above was added and incubated at room temperature (anti-M13-HRP as a secondary antibody). After tetramethylbenzidine (TMB) color development, OD650 was read detected by a microplate reader. The single clone was sequenced. The sequencing results of #8, #62, #125, and #137 showed the sequence characteristics of scFv. The 4 single clones were separately packaged with phage and precipitated with 5×PEG to increase the titer. the phage ELISA was verified with 10 uL phage (BSA, WT GFP, and pBpa GFP were used as the antigens, respectively), and the results were shown in Fig. 10.

The Hela cells transfected with pcDNA3.1-(TAC151TAG) GFP were blocked with 5% milk for 1 hour, incubated with #125 monoclonal phage library on ice for 30 min. 365 nm UV irradiation was applied for 30 min, followed by elution by acidic eluent for 30 min. After eluted with 0.1% sodium dodecyl sulfate (SDS) for 10 min and 20 min, respectively, 200 ug/mL trypsin were applied at 37°C for 30 min, and subjected to infected XL-Blue for 1 h followed by counting of single clones. The UV/non-UV ratio was shown in Table 9, indicating that the #125 phage displaying scFv was able to cross-link with the GFP epitope expressed on the cell membrane surface.

**Table 8: Output CFU ratio of natural human antibody phage library against pBpa-GFP with or without UV treatment**

| Sample | Coated antigen | UV-treated output CFU | Non-UV-treated output CFU | UV-treated output CFU / Non-UV-treated output CFU |
|---|---|---|---|---|
| Natural human antibody phage library | pBpa | 146 | 25 | 5.8 |

**Table 9: Output CFU ratio of #125 monoclonal phage library against pBpa-GFP under different elution times of 0.1% SDS with or without UV treatment**

| SDS elution time | UV-treated output CFU | Non-UV-treated output CFU | UV-treated output CFU/non-UV-treated output CFU |
|---|---|---|---|
| 10min | 194 | 5 | 38.8 |
| 20min | 153 | 0 | ∞ |

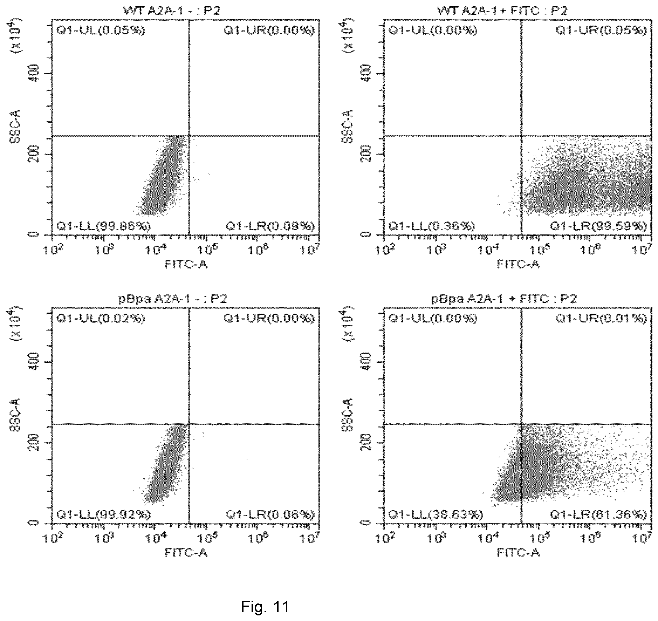

Next, this screening method was further applied to screen antibodies against multi-transmembrane protein A2A. pCDNA3.4-WT A2A and pCDNA3.4-(TTT168TAG)A2A eukaryotic expression plasmids (with a flag-tag at a N-terminal and a His-tag at a C-terminal) were constructed, and transfected into Hela cells, respectively. Flow cytometry after 48h of transfection showed the expression of both WT A2A and mutant A2A on the membrane (see Fig. 11). After that, the Hela cells expressing the mutant A2A (pBpa-A2A) were used for epitope-directed antibody screening: Hela cells expressing the mutant A2A (pBpa-A2A) were blocked with 5% milk for 1 hour and incubated with a human natural antibody phage library (the titer is 10¹²/mL) on ice for 30 min, followed by 365 nm UV irradiation for 30min; After eluting with an acid eluent for 30 min, followed by eluted with 0.1% SDS for 5 min, 10 min, 20 min, 25 min, and 30 min, respectively. Covalently bound phages were released by 200 ug/mL trypsin at 37°C for 30 min and subjected to infected with XL-Blue for 1 h, followed by the number of clones counting. The UV/non-UV ratio (see Table 10) was greater than 3 when eluted with 0.1% SDS for more than 20 min, indicating that these scFv-displaying phages were capable of cross-linking with target epitope. Single clones in UV-treated group eluted with 0.1% SDS for 25 min and 30 min, respectively were selected and amplified with primers pSEX-F and pSEX-R. PCR products larger than 750 bp were picked. 20 single clones were randomly selected for sequencing. Sequence alignment showed that monoclonal phages all exhibited typical scFv sequence features with diversity in CDR region. Phylogenetic tree analysis also showed the amino acid homology of these clones.

**Table 10: Output CUF ratios of natural human antibody phage library against pBpa-A2A under different elution times of 0.1% SDS with or without UV treatment**

| SDS elution time | UV-treated output CFU | Non-UV-treated output CFU | UV-treated output CFU / Non-UV-treated output CFU |
|---|---|---|---|
| 5min | 198 | 192 | 1.03 |
| 10min | 97 | 60 | 1.62 |
| 20min | 34 | 10 | 3.4 |
| 25min | 43 | 9 | 4.78 |
| 30min | 34 | 10 | 3.4 |

## Claims

1. A method for selecting antibodies against a specific epitope of a target antigen, wherein the method comprises the following steps:
(i) providing a support, wherein the support is immobilized with a mutant antigen formed by incorporation of amino acids with photocrosslinking activity in a target epitope of the target antigen;
(ii) providing conditions that enables the contact of the mutant antigen with antibodies in antibody display library, and applying light irradiation with suitable wavelength and energy to allow the mutant antigen to covalently cross-link with displayed antibodies in the library that binds to or near the target epitope to form antigen-antibody complexes;
(iii) performing elution under certain condition, wherein this condition enables the displayed antibodies that do not covalently cross-link with the mutant antigen to be washed away from the support, while the displayed antibodies that form the covalent cross-link remain on the support;
(iv)releasing the displayed antibodies that covalently cross-link with the mutant antigen from the support; and
(v) further selecting the displayed antibodies capable of binding to the target antigen from the displayed antibodies obtained in the step (iv).

2. The method according to claim 1, wherein the amino acids with the photocrosslinking activity is incorporated by genetic codon expansion.

3. The method according to claim 1, wherein the step (v) comprises repeated one or more rounds of the steps (i)-(iv).

4. The method according to claim 1, wherein the method further comprises sequencing the antibodies selected in the step (v).

5. The method according to claim 1, wherein the epitope is a linear epitope or a conformational epitope.

6. The method according to claim 1, wherein the antibody display library is selected from: IgG antibodies or antibody fragments such as Fab library, single chain Fv (scFv) library, or
the antibody display library is selected from: fully human antibody libraries, humanized antibody libraries, mouse immune antibody library, and synthetic or semi-synthetic antibody library designed based on antibody sequences of different species; or
a display carrier of the antibody display library is selected from: phages, bacteria, yeast, or mammalian cells; or
the antibody display library is a phage display antibody library.

7. The method according to claim 1, wherein the mutant antigen is a soluble protein, or a transmembrane protein expressed on a phospholipid membrane structure, wherein the mutant antigen can be directly immobilized on the support or indirectly immobilized on the support by membrane with phospholipid membrane structure.

8. The method according to claim 1, wherein the amino acids with photocrosslinking activity are a natural amino acid or a noncanonical amino acid.

9. The method according to claim 8, wherein the noncanonical amino acid photocrosslinking activity is selected from: p-benzoyl-L-phenylalanine (pBpa) or p-azido-L-phenylalanine (pAzF).

10. The method according to claim 9, wherein the light irradiation conditions suitable for pBpa cross-linking are: 365 nM, and 6 W.

11. The method according to claim 1, wherein the elution conditions of the step (iii) are selected from:
a) competitive elution with a buffer containing the target antigen;
b) acidic elution with a low-pH buffer; and
c) alkaline elution with a high-pH buffer.

12. The method according to claim 1, wherein the releasing of the step (iv) is by enzymatic digestion.

13. A method for selecting an antibody against a specific epitope of an antigen, wherein the method comprises:
(i) providing conditions that allows contact of mutant antigen with incorporation of amino acids with photocrosslinking activity in a target epitope with a labeled antibody display library to allows the antigen binding to the antibody, and applying light irradiation with suitable wavelength and energy to allow the mutant antigen to covalently cross-link to displayed antibodies in the library that binds to the target epitope to form antigen-antibody complex;
(ii) selecting the antigen-antibody complex that forms covalent cross-link with the mutant antigen, and releasing the displayed antibodies that form covalent cross-linking with the mutant antigen; and
(iii) further selecting the displayed antibodies capable of binding to the target antigen from the displayed antibodies obtained in the step (ii).

14. The method according to claim 13, wherein the mutant antigen contacts with the antibody display library in solution.

15. The method according to claim 14, wherein the mutant antigen is expressed on the cell surface; or wherein cells expressing the mutant antigen are selected by flow cytometry; or wherein the mutant antigen is a transmembrane protein.

## Patentansprüche

1. Verfahren zur Auswahl von Antikörpern gegen ein spezifisches Epitop eines Zielantigens, wobei das Verfahren die folgenden Schritte umfasst:
(i) Bereitstellung eines Trägers, auf dem ein mutiertes Antigen immobilisiert ist, das durch Einbau von Aminosäuren mit Photovernetzungsaktivität in ein Ziel-Epitop des Zielantigens gebildet wird;
(ii) Schaffung von Bedingungen, die den Kontakt des mutierten Antigens mit Antikörpern in einer Antikörper-Display-Bibliothek ermöglichen, und Anwendung von Lichtbestrahlung mit geeigneter Wellenlänge und Energie, um eine kovalente Vernetzung des mutierten Antigens mit den in der Bibliothek dargestellten Antikörpern zu ermöglichen, die an oder nahe dem Ziel-Epitop binden, um Antigen-Antikörper-Komplexe zu bilden;
(iii) Durchführung einer Elution unter bestimmten Bedingungen, wobei diese Bedingungen es ermöglichen, dass die dargestellten Antikörper, die nicht kovalent mit dem mutierten Antigen vernetzt sind, vom Träger abgewaschen werden, während die dargestellten Antikörper, die die kovalente Vernetzung bilden, auf dem Träger verbleiben;
(iv) Freisetzung der dargestellten Antikörper, die kovalent mit dem mutierten Antigen vernetzt sind, vom Träger; und
(v) weitere Auswahl der dargestellten Antikörper, die in der Lage sind, an das Zielantigen zu binden, aus den in Schritt (iv) erhaltenen dargestellten Antikörpern.

2. Verfahren nach Anspruch 1, wobei die Aminosäuren mit Photovernetzungsaktivität durch genetische Codon-Erweiterung eingebaut werden.

3. Verfahren nach Anspruch 1, wobei Schritt (v) ein oder mehrere Wiederholungen der Schritte (i)-(iv) umfasst.

4. Verfahren nach Anspruch 1, wobei das Verfahren ferner die Sequenzierung der in Schritt (v) ausgewählten Antikörper umfasst.

5. Verfahren nach Anspruch 1, wobei das Epitop ein lineares Epitop oder ein konformationelles Epitop ist.

6. Verfahren nach Anspruch 1, wobei die Antikörper-Display-Bibliothek ausgewählt ist aus: IgG-Antikörpern oder Antikörperfragmenten wie Fab-Bibliothek, Einzelkette-Fv (scFv)-Bibliothek, oder die Antikörper-Display-Bibliothek ist ausgewählt aus: vollständig humanen Antikörperbibliotheken, humanisierten Antikörperbibliotheken, Maus-immun-Antikörperbibliothek und synthetischen oder halbsynthetischen Antikörperbibliotheken, die auf Antikörpersequenzen verschiedener Spezies basieren; oder ein Display-Träger der Antikörper-Display-Bibliothek ist ausgewählt aus: Phagen, Bakterien, Hefen oder Säugetierzellen; oder die Antikörper-Display-Bibliothek ist eine Phagen-Display-Antikörperbibliothek.

7. Verfahren nach Anspruch 1, wobei das mutierte Antigen ein lösliches Protein oder ein Transmembranprotein ist, das auf einer Phospholipidmembranstruktur exprimiert wird, wobei das mutierte Antigen direkt auf dem Träger immobilisiert oder indirekt über eine Membran mit Phospholipidmembranstruktur auf dem Träger immobilisiert werden kann.

8. Verfahren nach Anspruch 1, wobei die Aminosäuren mit Photovernetzungsaktivität eine natürliche Aminosäure oder eine nicht-kanonische Aminosäure sind.

9. Verfahren nach Anspruch 8, wobei die Photovernetzungsaktivität der nicht-kanonischen Aminosäure ausgewählt ist aus: p-Benzoyl-L-Phenylalanin (pBpa) oder p-Azido-L-Phenylalanin (pAzF).

10. Verfahren nach Anspruch 9, wobei die Lichtbestrahlungsbedingungen, die für die pBpa-Vernetzung geeignet sind, Folgendes betragen: 365 nm und 6 W.

11. Verfahren nach Anspruch 1, wobei die Elutionsbedingungen in Schritt (iii) ausgewählt sind aus:
a) kompetitive Elution mit einem Puffer, der das Zielantigen enthält;
b) saure Elution mit einem Puffer mit niedrigem pH-Wert; und
c) alkalische Elution mit einem Puffer mit hohem pH-Wert.

12. Verfahren nach Anspruch 1, wobei die Freisetzung in Schritt (iv) durch enzymatische Verdauung erfolgt.

13. Verfahren zur Auswahl eines Antikörpers gegen ein spezifisches Epitop eines Antigens, wobei das Verfahren umfasst:
(i) Schaffung von Bedingungen, die den Kontakt eines mutierten Antigens mit eingebauten Aminosäuren mit Photovernetzungsaktivität in einem Ziel-Epitop mit einer markierten Antikörper-Display-Bibliothek ermöglichen, um die Bindung des Antigens an den Antikörper zu ermöglichen, und Anwendung von Lichtbestrahlung mit geeigneter Wellenlänge und Energie, um eine kovalente Vernetzung des mutierten Antigens mit den in der Bibliothek dargestellten Antikörpern zu ermöglichen, die an das Ziel-Epitop binden, um einen Antigen-Antikörper-Komplex zu bilden;
(ii) Auswahl des Antigen-Antikörper-Komplexes, der eine kovalente Vernetzung mit dem mutierten Antigen bildet, und Freisetzung der dargestellten Antikörper, die eine kovalente Vernetzung mit dem mutierten Antigen bilden; und
(iii) weitere Auswahl der dargestellten Antikörper, die in der Lage sind, an das Zielantigen zu binden, aus den in Schritt (ii) erhaltenen dargestellten Antikörpern.

14. Verfahren nach Anspruch 13, wobei das mutierte Antigen in Lösung mit der Antikörper-Display-Bibliothek in Kontakt kommt.

15. Verfahren nach Anspruch 14, wobei das mutierte Antigen auf der Zelloberfläche exprimiert wird; vorzugsweise werden Zellen, die das mutierte Antigen exprimieren, durch Durchflusszytometrie ausgewählt; vorzugsweise ist das mutierte Antigen ein Transmembranprotein.

## Revendications

1. L'invention concerne une méthode de sélection des anticorps contre un épitope spécifique d'un antigène cible, comprenant les étapes suivantes :
(i) fournir un support, sur lequel est immobilisé un antigène mutant formé par incorporation d'acides aminés contenant des groupes fonctionnels à photoréticulation dans un épitope cible de l'antigène cible ;
(ii) fournir des conditions permettant la mise en contact d'un antigène mutant avec les anticorps présents dans la bibliothèque d'anticorps exprimés sur des phages (ci-après dénommés « lesdits anticorps »), puis appliquer une radiation lumineuse à longueur d'onde et puissance appropriées permettant une réticulation par liaisons covalentes entre un antigène mutant et lesdits anticorps qui se lient à l'épitope cible ou dirigent vers celui-ci pour la formation d'un complexe antigène-anticorps ;
(iii) procéder à l'élution dans certaines conditions où lesdits anticorps ayant subi une réticulation par liaisons covalentes avec un antigène mutant restent fixé sur le support, tandis que les autres anticorps sont éliminés du support par un lavage ;
(iv) libérer du support lesdits anticorps ayant subi une réticulation par liaisons covalentes avec un antigène mutant ; et
(v) sélectionner, parmi lesdits anticorps récupérés à l'étape (iv), ceux qui sont capables de se fixer sur l'antigène cible.

2. Méthode selon la revendication 1, **caractérisée en ce que** lesdits acides aminés contenant des groupes fonctionnels à photoréticulation sont incorporés par l'expansion du code génétique.

3. Méthode selon la revendication 1, **caractérisée en ce que** l'étape (v) comprend la répétition d'un ou plusieurs tours des étapes (i) à (iv).

4. Méthode selon la revendication 1, **caractérisée en ce que** ladite méthode contient également le séquençage des anticorps sélectionnés à l'étape (v).

5. Méthode selon la revendication 1, **caractérisée en ce que** ledit épitope est un épitope non séquentiel ou un épitope de conformation.

6. Méthode selon la revendication 1, **caractérisée en ce que** ladite bibliothèque d'anticorps exprimés sur des phages est sélectionnée parmi : les IgG, les fragments d'anticorps tels que le fragment Fab et le fragment variable à chaîne unique (scFv), les anticorps entièrement humains, les anticorps humanisés, les anticorps monoclonaux de souris et de rat, les anticorps synthétiques ou semi-synthétiques conçus sur la base de séquences d'anticorps de différentes espèces ; les porteurs utilisés pour lesdits anticorps sont sélectionnés parmi : les phages, les bactéries, les levures ou les cellules de mammifères.

7. Méthode selon la revendication 1, **caractérisée en ce que** ledit antigène mutant est une protéine soluble ou une protéine transmembranaire exprimée sur la membrane de phospholipide, dans laquelle ledit antigène mutant peut être directement immobilisé sur le support ou indirectement par la membrane de phospholipide.

8. Méthode selon la revendication 1, **caractérisée en ce que** lesdits acides aminés contenant des groupes fonctionnels à photoréticulation sont des acides aminés naturels ou des acides aminés non-canoniques.

9. Méthode selon la revendication 8, **caractérisée en ce que** lesdits acides aminés non-canoniques contenant des groupes fonctionnels à photoréticulation sont sélectionnés parmi : le *p*-benzoyle-L-phénylalanine (pBpa) ou le *p*-azido-L-phénylalanine (pAzF).

10. Méthode selon la revendication 9, **caractérisée en ce que** les conditions de radiation lumineuse adaptées à la réticulation du pBpa sont : 365 nM et 6 W.

11. Méthode selon la revendication 1, **caractérisée en ce que** lesdites conditions d'élution à l'étape (iii) sont sélectionnées parmi :
a) une élution compétitive avec un tampon contenant l'antigène cible ;
b) une élution acide avec un tampon à faible pH ; et
c) une élution alcaline avec un tampon à pH élevé.

12. Méthode selon la revendication 1, **caractérisée en ce que** lesdits anticorps à l'étape (iv) sont libérés par l'élution enzymatique.

13. L'invention concerne une méthode de sélection des anticorps contre un épitope spécifique d'un antigène cible, comprenant les procédés suivants :
(i) fournir des conditions permettant la mise en contact d'un antigène mutant, formé par incorporation d'acides aminés contenant des groupes fonctionnels à photoréticulation dans un épitope cible, avec un anticorps présent dans la bibliothèque d'anticorps exprimés sur des phages, afin que l'anticorps puisse se fixer sur l'antigène, puis appliquer une radiation lumineuse à longueur d'onde et puissance appropriées permettant une réticulation par liaisons covalentes entre un antigène mutant et lesdits anticorps qui se lient à l'épitope cible pour la formation d'un complexe antigène-anticorps ;
(ii) sélectionner le complexe antigène-anticorps réticulé par liaisons covalentes avec l'antigène mutant, et libérer lesdits anticorps réticulés par liaisons covalentes avec l'antigène mutant ; et
(iii) sélectionner, parmi lesdits anticorps récupérés à l'étape (ii), ceux qui sont capables de se fixer sur l'antigène cible.

14. Méthode selon la revendication 13, **caractérisée en ce que** ledit antigène mutant est mis en contact avec lesdits anticorps dans une solution.

15. Méthode selon la revendication 14, **caractérisée en ce que** ledit antigène mutant est exprimé à la surface des cellules ; de préférence, les cellules exprimant ledit antigène mutant sont triées par la cytométrie en flux ; de préférence, ledit antigène mutant est une protéine transmembranaire.
